(19) Europäisches Patentamt
European Patent Office
Office européen des brevets

(11) Veröffentlichungsnummer: **0 283 438 A2**

(12) **EUROPÄISCHE PATENTANMELDUNG**

(21) Anmeldenummer: **88810157.3**

(22) Anmeldetag: **14.03.88**

(51) Int. Cl.⁴: **C 07 D 213/53**
A 01 N 43/40

(30) Priorität: **18.03.87 CH 1028/87**
**09.04.87 CH 1361/87**

(43) Veröffentlichungstag der Anmeldung:
**21.09.88 Patentblatt 88/38**

(84) Benannte Vertragsstaaten:
**AT BE CH DE FR GB IT LI NL**

(71) Anmelder: **CIBA-GEIGY AG**
**Klybeckstrasse 141**
**CH-4002 Basel (CH)**

(72) Erfinder: **Farooq, Saleem, Dr.**
**Kirchackerstrasse 27**
**CH-4422 Arisdorf (CH)**

(54) **Pyridyl-diazabutadienyldisulfide.**

(57) Neue gegebenenfalls substituierte Pyrid-3-yl-2,3-diazabutadien-1-yl]-disulfide der Formel

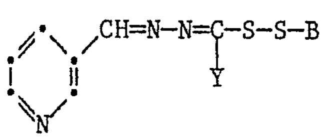

(I)

worin
B unsubstituiertes oder substituiertes $C_1$-$C_6$-Alkyl oder unsubstituiertes oder substituiertes Phenyl, dem Rest

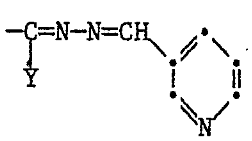

und
Y unsubstituierten oder substituierten aromatischen Carbo- oder Heterocyclus; sowie deren Salze und optischen Isomeren; Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen weisen insbesondere gute Wirksamkeit gegen pflanzenschädigende saugende Insekten auf.

EP 0 283 438 A2

Bundesdruckerei Berlin

**Beschreibung**

Pyridyl-diazabutadienyldisulfide

Die vorliegende Erfindung betrifft neue insektizid wirkame Pyridyl-diazabutadienyldisulfide, Verfahren zu ihrer Herstellung, Mittel, welche diese Pyridyl-diazabutadienyldisulfide enthalten und ihre Verwendung in der Schädlingsbekämpfung.

Die erfindungsgemässen Pyridyl-diazabutadienyldisulfide entsprechen der Formel I

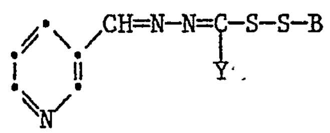

(I)

worin

B unsubstituiertes oder substituiertes $C_1$-$C_6$-Alkyl, unsubstituiertes oder substituiertes Phenyl, oder den Rest

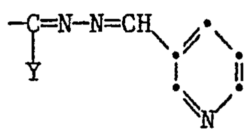

und

Y unsubstituierten oder substituierten aromatischen Carbo- oder Heterocyclus bedeuten, sowie den Salzen einer Verbindung der Formel I.

Von den erfindungsgemässen Verbindungen werden jene der Formel Ia

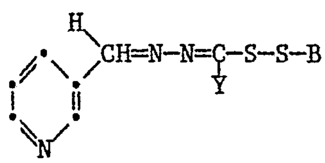

(Ia),

worin

Y einen unsubstituierten oder substituierten aromatischen Carbo-oder Heterocyclus, und

B unsubstituiertes oder substituiertes $C_1$-$C_6$-Alkyl oder unsubstituiertes oder substituiertes Phenyl bedeuten, bevorzugt.

Unter den Verbindungen der Formel Ia stehen wegen ihrer insektiziden Wirkung diejenigen im Vordergrund, bei welchen Y Pyridyl oder unsubstituiertes oder substituiertes Phenyl, B $C_1$-$C_6$-Alkyl, ein-oder mehrfach halogeniertes $C_1$-$C_6$-Alkyl, $C_1$-$C_4$-Alkoxy-$C_1$-$C_4$-alkyl, $C_1$-$C_4$-Alkylthio-$C_1$-$C_6$-alkyl, Cyano-$C_1$-$C_6$-alkyl, Nitro-$C_1$-$C_4$-alkyl oder den Rest

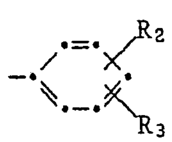

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeuten.

Dabei sind diejenigen dieser Verbindungen bevorzugt, bei welchen Y Pyridyl oder den Rest

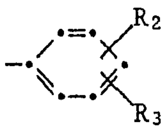

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeutet und
B Cyano-$C_1$-$C_4$-alkyl bedeutet.

Insbesondere bevorzugt ist davon das [1-(4-Chlorphenyl)-4-(pyrid-3-yl)-2,3-diazabutadien-1-yl]-(1,1-dimethyl-1-cyanomethyl) disulfid.

Von den Verbindungen der Formel Ia stehen ausserdem jene im Vordergrund, bei welchen
Y Pyridyl oder den Rest

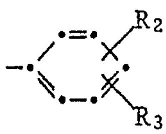

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeutet und
B Phenyl, Nitrophenyl, 4-Chlorphenyl oder 4-Tolyl bedeutet.

Besondere Erwähnung verdienen davon jene Verbindungen, bei welchen Y 4-Chlorphenyl bedeutet.

Von den erfindungsgemässen Verbindungen werden auch jene der Formel Ib

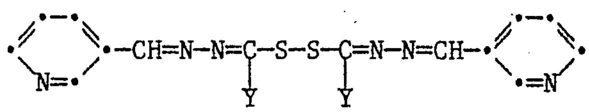

(Ib),

worin
Y einen Rest

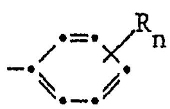

oder

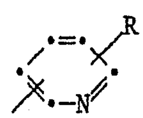

bedeutet, wobei
R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl substituiertes Phenyl, Phenyl-$C_1$-$C_3$-alkyl, Phenoxy, Phenylthio oder Pyridyloxy bedeutet und
n für eine Zahl von 1 bis 5 steht, einschliesslich der Salze bevorzugt.

Bevorzugt werden erfindungsgemäss Verbindungen der Formel Ib, worin
R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen oder $C_1$-$C_4$-Halogenalkylthio mit 1 bis 9 Halogenatomen bedeutet und n für eine Zahl von 1 bis 3 steht.

Weiterhin bevorzugt werden erfindungsgemäss Verbindungen der Formel Ib, worin
R Wasserstoff, $C_1$-$C_4$-Alkyl, Fluor, Chlor, Brom, Nitro, $C_1$-$C_4$-Alkoxy oder Trifluormethyl bedeutet.

Von besonderem Interesse sind solche erfindungsgemässen Verbindungen der Formel Ib worin
n für eine Zahl 1 oder 2 steht, sowie solche, worin
Y den Rest

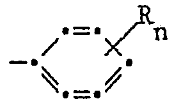

bedeutet, wobei sich mindestens ein Rest R in 4-Stellung befindet.

Unter dem Begriff Alkyl selbst oder als Bestandteil eines anderen Substituenten sind geradkettige oder verzweigte und je nach Zahl der angegebenen Kohlenstoffatome im Rahmen der vorliegenden Erfindung beispielsweise folgende Gruppen zu verstehen: Methyl, Ethyl, Propyl, Butyl, sowie ihre Isomeren, wie Isopropyl, Isobutyl, tert.-Butyl, sek.-Butyl.

Unter dem Begriff Halogenalkyl im Rahmen der vorliegenden Erfindung sind Alkylreste, wie Methyl, Aethyl, n-Propyl, und Isopropyl, Butyl usw. zu verstehen, die mit 1 bis 9 unterschiedlichen oder gleichartigen Halogenatomen substituiert sind, wobei es sich um perhalogenierte Alkylreste aber auch um solche handeln kann, deren Wasserstoffatome nur teilweise mit Halogen substituiert sind.

Unter dem Begriff Halogen im Rahmen der vorliegenden Erfindung ist Fluor, Chlor und Brom zu verstehen, vorzugsweise Fluor und Chlor.

Bei den als Substituenten in Betracht kommenden carbocyclischen oder heterocyclischen Arylen handelt es sich um unsubstituiertes oder substituiertes Phenyl, 1- oder 2-Napthtyl und um Pyridyl.

Der vorliegende Erfindungsgegenstand umfasst auch die Salze, insbesondere die pflanzenphysiologisch umbedenklichen Salze, der Verbindungen der Formel I. Als derartige Salze mit organischen und anorganischen Säuren kommen z.B. die folgenden in Betracht: Chloride, Bromide, Jodide, Sulfate, Hydrogensulfate, Chlorate, Perchlorate, Rhodanide, Nitrate, Phosphate, Hydrogenphosphate, Tetrafluorborate, Formiate, Acetate, Trichloracetate, Trifluoracetate, Phenylsulfonate, Oxalate, Malonate, Succinate, Malate, Tartrate oder Citrate.

Die Verbindungen der Formel 1a können dadurch hergestellt werden, dass man ein 5-Pyridyl-4,5-dihydrothiadiazol der Formel II

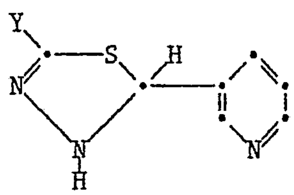

(II)

zunächst mit einer Base, dann mit einem Sulfenylchlorid der Formel III,

Cl-S-B (III),

umsetzt, wobei Y und B die anfangs angegebene Bedeutung haben und das erwünschte Produkt gegebenenfalls in einer seiner Salze überführt und isoliert.

Dieses Verfahren ist neu und bildet ebenfalls den Gegenstand der vorliegenden Erfindung.

Die Sulfenylchloride der Formel III sind bekannte und im Chemikalienhandel erhältliche Verbindungen, deren Herstellung nach mehreren Methoden aus der Literatur bekannt ist. Eine Uebersicht über die Herstellungsverfahren der Sulfenylchloride ist in der Zeitschrift "Synthesis", 561-580 (1970) veröffentlicht worden.

Bei der erfindungsgemässen Herstellung der Pyridyl-diazabutadienylsulfide der Formel Ia können die eingesetzten Dihydrothiadiazole der Formel II sowohl mit anorganischen Basen, wie z.B. Kalium- oder Natriumhydroxyd, Kalium- oder Natriumcarbonat, als auch mit organischen Basen, wie z.B. Trialkylaminen, umgesetzt werden.

Das erfindungsgemässe Verfahren zur Herstellung der Verbindungen der Formel Ia wird vorzugsweise in einem Lösungsmittel vorgenommen. Geeignete Lösungsmittel sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid; Dimethylsulfoxid und andere Lösungsmittel, die die Reaktion nicht beeinträchtigen. Diese Lösungsmittel können auch als Gemische verwendet werden. Die Reaktionstemperatur kann in einem weiten Bereich von -10 bis +200°C liegen. Bevorzugt wird ein Temperaturbereich von Raumtemperatur bis etwa 150°C.

Die Verbindungen der Formel Ib können hergestellt werden, indem man eine Verbindung der Formel II, vorzugsweise in Gegenwart einer basischen Substanz oxydiert. Als Oxydationsmittel kommen z.B. Chlor, Jod oder Sauerstoff in Frage. Basische Substanzen, die bei dem Verfahren Verwendung finden können, sind z.B. Alkali- und Erdalkalihydroxide oder -carbonate, wie z.B. KOH, NaOH, $K_2CO_3$, $Na_2CO_3$ usw.

Auch das zu den erfindungsgemässen Verbindungen der Formel Ib führende Verfahren wird vorzugsweise in einem Lösungsmittel vorgenommen. Die Reaktionstemperatur kann in einem weiten Bereich von -10 bis

+100°C liegen. Bevorzugt wird ein Temperaturbereich von etwa 0° bis 60°C.

Geeignete Lösungsmittel bei der Herstellung der Verbindungen der Formel I sind z.B. aromatische Kohlenwasserstoffe, wie Benzol, Toluol und Xylol; Ketone, wie Aceton, Cyclohexanon und Methyläthylketon; Aether, wie Tetrahydrofuran, Dioxan und Diäthyläther; halogenierte Kohlenwasserstoffe, wie Chloroform, Tetrachlorkohlenstoff und Chlorbenzol; Alkohole, wie Aethanol und Propanol; Ester aliphatischer Säuren, wie Aethylacetat; aliphatische Amide, wie Dimethylformamid und Dimethylacetamid; Dimethylsulfoxid und andere Lösungsmittel, die die Reaktion nicht beeinträchtigen. Diese Lösungsmittel können auch als Gemische verwendet werden.

Die Ausgangsverbindungen der Formel II können in an sich bekannter Weise (vgl. D.M. Evans et al., J.Chem.Soc., Chem. commun. 1982, 188; K.N. Zelenin et al., Khim. Geterotsikl. Soedin, 1982 No. 7, 904) hergestellt werden durch Umsetzung einer Verbindung der Formel IV

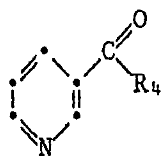

(IV),

mit einer Verbindung der Formel V

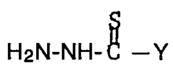

(V),

wobei in den Verbindungen IV und V $R_4$ $C_1$-$C_4$-Alkyl und Y die vorstehend unter Formel Ib angegebenen Bedeutungen haben.

Die Verbindungen der Formel II können weiterhin gemäss der europäischen Patentanmeldung Nr. 0 207 004 dadurch hergestellt werden, dass man eine Verbindung der Formel VI

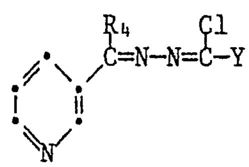

(VI),

worin $R_4$ und Y die vorstehend angegebenen Bedeutungen haben, mit einem Sulfid umsetzt.

Es wurde überraschenderweise gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tieren befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen entspricht einer Abtötungsrate (Mortalität) von mindestens 50-60 % der erwähnten Schädlinge.

Mit Hilfe der erfindungsgemässen Verbindungen der Formel I können vor allem pflanzenschädigende Insekten, speziell pflanzenschädigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische, vor allem aber durch Kontakt-Wirkung gegen saugende Insekten, insbesondere gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formel I zeichnen sich weiterhin durch eine gute Wirkung gegen larvale Insektenstadien und Nymphen, speziell von fressenden Schadinsekten, aus. Insbesondere können die Verbindungen der Formel I mit ausgezeichnetem Erfolg gegen pflanzenschädigende Zikaden, speziell in Reis-Kulturen, eingesetzt werden.

Die Verbindungen eignen sich ferner zur Bekämpfung von Ektoparasiten, z.B. Lucilia sericata, sowie von Zecken an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische

Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokonuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthaltend vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol ewähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Diese Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag Münschen/Wien 1981.

Die pestiziden Zubereitungen enthalten - auf das Gewicht bezogen -in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von Ausgangsverbindungen:

a) Herstellung von 2-(4-Chlorphenyl)-5-(pyrid-3-yl)-4,5-dihydro-1,3,4-thiadiazol

Es werden 39,2 g p-Chlorthiobenzoesäure-hydrazid in 350 ml Aethanol gelöst. Zu dieser Lösung werden unter Stickstoff 21,4 g Pyridin-3-aldehyd (gelöst in 20 ml Aethanol) zugetropft. Die Reaktionstemperatur wird durch Kühlung auf 20° C gehalten. Nach Beendigung der Zugabe wird während 1 Stunde bei Rückflusstemperatur nachgerührt und anschliessend das Reaktionsgemisch durch feinteilige Diatomeenerde filtriert. Das Filtrat wird auf Dreiviertel seines Anfangsvolumens eingeengt und das ausgefallene Produkt abfiltriert, mit Hexan gewaschen und getrocknet. Man erhält die Titelverbindung der Formel

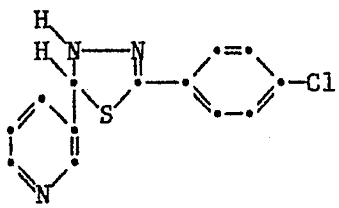

mit einem Smp. von 106-108° C.

b) Herstellung von 2-(3-Nitrophenyl)-5-(pyrid-3-yl)-4,5-dihydro-1,3,5-thiadiazol

Eine Lösung von 8,7 g Kaliumhydroxyd in 100 ml Aethanol wird mit Schwefelwasserstoff gesättigt. Zu dieser Lösung werden 8,7 g Kaliumhydroxyd in 100 ml Aethanol zugetropft. Anschliessend wird die erhaltene Lösung mit 22,4 g 1-Chlor-1-(3-nitrophenyl)-4-(3-pyridyl)-2,3-diazabutadien portionsweise unter Eiskühlung versetzt. Das Reaktionsgemisch wird während 1 Stunde bei Raumtemperatur nachgerührt, dann eingeengt und der Rückstand in Essigester aufgenommen. Die Essigester-Lösung wird zweimal mit Wasser und zweimal mit gesättigter Kochsalzlösung gewaschen, über Natriumsulfat getrocknet, filtriert und eingedampft.

Man erhält auf diese Weise die Titelverbindung der Formel

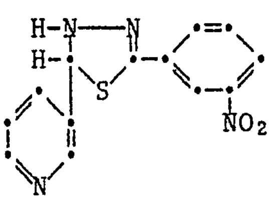

mit einem Schmelzpunkt von 109-111° C

Beispiel 2: Herstellung

1-(4-Chlorphenyl-4-(pyrid-3-yl)-2,3-diazabutadien-1-yl-1,1-dimethyl-1-cyanomethyldisulfid.

Zur vorgelegten Lösung von 13,8 g (0,05M) 2-(4-Chlorphenyl)-5-(pyrid-3-yl)-4,5-dihydrothiazol (EP-A 207 004) in 80 ml Tetrahydrofuran werden 6,1 g (0,06M) Triäthylamin gegeben und die Lösung wird auf 0 - 5° C abgekühlt. Hierzu wird während 10 Minuten 6,8 g (0,05M) α-(Chlorthio)-isobutyronitril, gelöst in 10 ml Tetrahydrofuran getropft. Das entstandene Reaktionsgemisch 5 Stunden lang bei 20-25° C gerührt, filtriert und die Mutterlauge in Vakuum bei 60° verdampft. Der Verdampfungsrückstand wird in 200 ml Aethylacetat gelöst, die Lösung mit je 20 ml Wasser und 20 ml gesättigter Kochsalzlösung gewaschen, über Natriumsulfat

getrocknet, filtriert und im Vakuum bei 60° verdampft.

Das als Rückstand erhältliche Rohprodukt wird in 200 ml Aethylacetat erneut aufgelöst, die kalte Lösung über Kieselgel filtriert und das Lösungsmittel erneut verdampft.

Der Rückstand besteht aus dem erwünschten 1-(4-Chlorphenyl-4-(pyrid-3-yl)-2,3-diazabutadien-1-yl-1,1-dimethyl-1-cyanomethyldisulfid in gereinigter Form, mit einem Schmelzpunkt von 83-85°C.

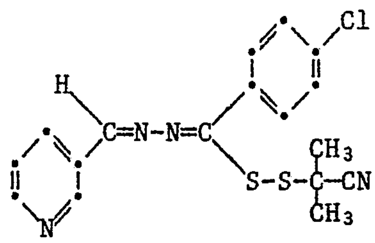

Auf analoge Weise werden die folgenden Verbindungen der Formel Ia hergestellt:

Tabelle 1

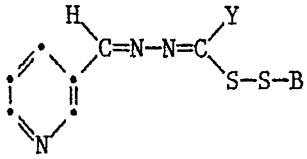

(Ia),

| Verbindung Nr. | Y | B | physikalische Daten |
|---|---|---|---|
| 1 | $4\text{-}Cl\text{-}C_6H_4\text{-}$ | $-C(CH_3)_2CN$ | Smp. 83-85°C |
| 2 | $4\text{-}CH_3\text{-}C_6H_4\text{-}$ | $-C(CH_3)_2CN$ | Smp. 78-79°C |
| 3 | $4\text{-}CF_3\text{-}C_6H_4\text{-}$ | $-C(CH_3)_2CN$ | Smp. 82-84°C |
| 4 | $4\text{-}CH_3O\text{-}C_6H_4\text{-}$ | $-C(CH_3)_2CN$ | $n_D^{20}$: 1,6569 |
| 5 | $4\text{-}F\text{-}C_6H_4\text{-}$ | $-C(CH_3)_2CN$ | Smp. 97-98°C |
| 6 | $4\text{-}Br\text{-}C_6H_4\text{-}$ | $-C(CH_3)_2CN$ | $n_D^{20}$: 1,6608 |
| 7 | $3\text{-}CH_3\text{-}C_6H_4\text{-}$ | $-C(CH_3)_2CN$ | |
| 8 | $3\text{-}CF_3\text{-}C_6H_4\text{-}$ | $-C(CH_3)_2CN$ | $n_D^{20}$: 1,5964 |
| 9 | $3\text{-}Cl\text{-}C_6H_4\text{-}$ | $-C(CH_3)_2CN$ | Smp. 66-68°C |
| 10 | $3,4\text{-}Cl_2\text{-}C_6H_3$ | $-C(CH_3)_2CN$ | Smp. 126-128°C |
| 11 | $2,4\text{-}Cl_2\text{-}C_6H_3\text{-}$ | $-C(CH_3)_2CN$ | |
| 12 | 3-Pyridyl- | $-C(CH_3)_2CN$ | Smp. 92-94°C |
| 13 | 2-Pyridyl- | $-C(CH_3)_2CN$ | |
| 14 | 4-Pyridyl- | $-C(CH_3)_2CN$ | |
| 15 | $4\text{-}Cl\text{-}C_6H_4\text{-}$ | $4\text{-}NO_2\text{-}C_6H_4\text{-}$ | |
| 16 | $4\text{-}Cl\text{-}C_6H_4\text{-}$ | $3\text{-}NO_2\text{-}C_6H_4\text{-}$ | |
| 17 | $4\text{-}Cl\text{-}C_6H_4\text{-}$ | $2\text{-}NO_2\text{-}C_6H_4\text{-}$ | |
| 18 | $4\text{-}Cl\text{-}C_6H_4\text{-}$ | $C_6H_5\text{-}$ | |
| 19 | $4\text{-}Cl\text{-}C_6H_4\text{-}$ | $4\text{-}Cl\text{-}C_6H_4\text{-}$ | |
| 20 | $4\text{-}Cl\text{-}C_6H_4\text{-}$ | $4\text{-}CH_3\text{-}C_6H_4\text{-}$ | Smp. 104-106°C |

Beispiel 3: Herstellung von Bis[1-(4-Chlorphenyl)-4-(pyrid-3-yl)-2,3-diazabutadien-1-yl]-disulfid

Zu einer auf 0°C gekühlten Lösung von 15 g des nach Beispiel 1a) erhaltenen 2-(4-Chlorphenyl)-5-(pyrid-3-yl)-4,5-dihydro-1,3,4-thiadiazols in 80 ml Aceton werden 3,6 g Kaliumhydroxid-Pulver zugegeben. Nach ca. 5 Minuten Rühren bei 0° bis 5°C werden 6,9 g Jod portionsweise hinzugefügt und die exotherme Reaktion wird durch Eiskühlung bei etwa 0° bis 5°C gehalten. Anschliessend wird der Ansatz während 4 Stunden bei ca. 0°C gerührt. Das ausgefallene Rohprodukt wird abfiltriert und mit wenig Aceton nachgewaschen. Das Rohprodukt wird in Tetrahydrofuran:Essigester (1:3) aufgenommen, einmal mit Wasser und zweimal mit gesättigter Kochsalzlösung gewaschen, über Natriumsulfat getrocknet und dann eingedampft. Die so erhaltene Titelverbindung der Formel

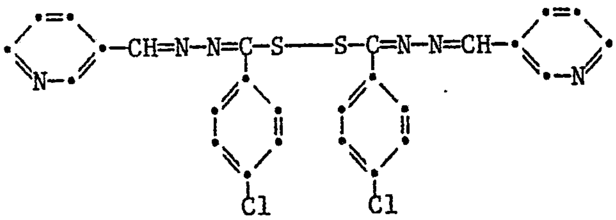

weist einen Schmelzpunkt von 143-145°C auf (Verbindung Nr. 21).

In analoger Weise wie oben angegeben ist auch die folgende Verbindung erhältlich:

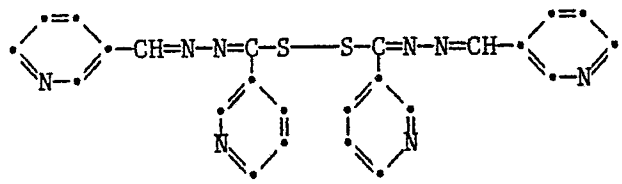

Smp. 105° - 108°C (Verbindung Nr. 22).

Wie vorstehend beschrieben sind auch die folgenden Verbindungen der Formel Ib erhältlich, in denen Y die folgenden Bedeutungen hat:

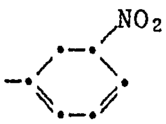

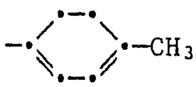

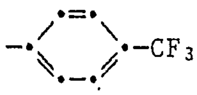

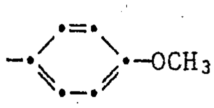

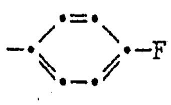

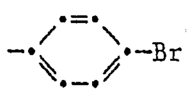

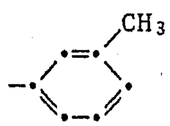

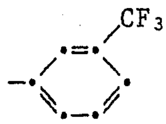

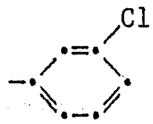

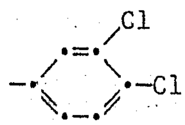

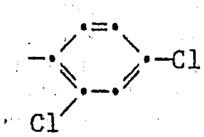

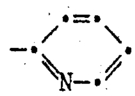

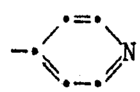

Beispiel 4:

Formulierungen für Wirkstoffe der Formeln I gemäss Beispiele 2 und 3 resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |

| | | | |
|---|---|---|---|
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentrationen verdünnen lassen.

2. Emulsions-KonzentratWirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-GranulatWirkstoff oder Wirkstoffkombination 10 %
Na-Ligninsulfonat 2 %
Carboxymethylcellulose 1 %
Kaolin 87%

Der Wirkstoff wird mit dem Zusatzstoffen vermischt, vermahlen und mit Wasser angefeuchtet. Dieses Gemisch wird extrudiert, granuliert und anschliessend im Luftstrom getrocknet.

5. Umhüllungs-GranulatWirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-KonzentratWirkstoff oder Wirkstoffkombination 40 %
Aethylenglykol 10 %
Nonylphenolpolyäthylenglykoläther (15 Mol AeO) 6%
Na-Ligninsulfonat 10 %
Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder

gewünschten Konzentration hergestellt werden können.

Biologische Beispiele

Beispiel 5.1: Wirkung gegen Musca domestica

Je 50 g frisch zubereitetes CSMA-Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispielen 2 und 3 zeigen gute Wirkung im obigen Test.

Beispiel 5.2: Wirkung gegen Lucilia sericata (Larven)

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss den Beispielen 2 und 3 zeigen in diesem Test gute Wirkung gegen Lucilia sericata.

Beispiel 5.3: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 200 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I gemäss den Beispielen 2 und 3 zeigen gute Wirkung im obigen Test.

Beispiel 5.4: Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend die zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer rel. Luftfeuchtigkeit von etwa 55 % durchgeführt.

Eine Mortalität von 80-100 % in diesem Test ergibt die Verbindung Nr. 1 gemäss Beispiel 2 bei 400 ppm. Auch die Verbindungen der Formel V gemäss Beispiel 3 zeigen gute Wirkung in diesem Test.

Beispiel 5.5: Systemische Wirkung auf Aphis craccivora (Boden)

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25 %igen Spritzpulver) in einer Konzentration von 800 ppm direkt auf die Erde in den Töpfen.

Nach 24 Stunden werden auf die oberirdischen Pflanzenteile Blattläuse der Spezies Aphis craccivora gesetzt und die Pflanzen mit einem Plastikzylinder überstülpt, um die Läuse vor einer eventuellen direkten Kontakt- oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet, Der Versuch wird bei 25°C und ca. 70 % relativer Luftfeuchtigkeit durchgeführt.

Verbindungen der Formel I gemäss den Beispielen 2 und 3 zeigen gute Wirkung in diesem Test.

Beispiel 5.6: Kontaktwirkung auf Myzus persicae

Etwa 4 bis 5 Tage alte, in Wasser angezogene Erbsenkeimlinge (Vicia faba) werden vor Versuchsbeginn jeweils mit ca. 200 Individuen der Spezies Myzus persicae besiedelt. Die so behandelten Pflanzen werden 24 Stunden später mit einer wässrigen Suspension enthaltend bis zu 200 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 und 72 Stunden nach Applikation. Der Versuch wird bei 21-22°C und etwa 60 % relativer Luftfeuchtigkeit durchgeführt.

Verbindung Nr. 1 gemäss Beispiel 2 sowie die Verbindung Nr. 21 gemäss Beispiel 3 ergeben bei 12,5 ppm jeweils eine 80-100 %ige Mortalität in diesem Test.

Beispiel 5.7: Systemische Wirkung auf Myzus persicae

Bewurzelte Kohlpflanzen im 4- bis 5-Blatt-Stadium werden in Töpfe, welche 60 ccm Erde enthalten, verpflanzt. Ansschliessend werden 50 ml einer wässrigen Formulierung der zu prüfenden Verbindung (erhalten aus einem 25%igen Spritzpulver) jeweils in einer Konzentration von 800 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen direkten Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25°C und 60% relativer Luftfeuchtigkeit durchgeführt.

Verbindungen der Formel I gemäss den Beispielen 2 und 3 zeigen gute Wirkung in obigem Test.

Beispiel 5.8: Blattpenetrations-Wirkung auf Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen. Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60% relativer Luftfeuchtigkeit durchgeführt. Die Auswertung auf %-Mortalität erfolgt 48 Stunden nach Wirkstoffapplikation.

Verbindungen der Formel I gemäss den Beispielen 2 und 3 zeigen gute Wirkung in obigem Test.

Beispiel 5.9: Frassgift-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 800 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss den Beispielen 2 und 3 zeigen gute Wirkung in diesem Test.

Beispiel 5.10: Ovizide Wirkung auf Laodelphax striatellus und Nilaparavata lugens;

Der Test wird an wachsenden Pflanzen durchgeführt. Jeweils 4 Reispflanzen (Dicke des Stengels 8 mm, Höhe ca. 20 cm) werden in Töpfe (Durchmesser von 8 cm) eingepflanzt.

Die Pflanzen werden auf einem Drehteller mit 100 ml einer acetonischen Lösung enthaltend 400 ppm des jeweiligen Wirkstoffes besprüht. Nach dem Antrocknen des Spritzbelages wird jede Pflanze mit 3 adulten Weibchen besiedelt. Um die Tiere am Entweichen zu hindern, wird über jede besiedelte Pflanze ein Glaszylinder gestülpt und dieser mit einem Gaze-Deckel abgedeckt. Die Weibchen bleiben 4 Tage zur Eiablage auf der behandelten Pflanze und werden dann entfernt.

Etwa 8 Tage nach Besiedelung schlüpfen die jungen Zikaden, und es erfolgt die Auszählung. Aus dem Vergleich der Anzahl geschlüpfter Larven auf den behandelten Pflanzen zu den auf unbehandelten Kontrollpflanzen geschlüpften Tieren wird die prozentuale Mortalität bestimmt.

Verbindungen der Formel I gemäss den Beispielen 2 und 3 zeigen in obigem Test gute ovizide Wirkung.

**Patentansprüche**

1. Verbindungen der Formel I

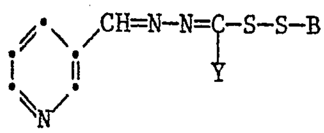

(I)

worin
B unsubstituiertes oder substituiertes $C_1$-$C_6$-Alkyl oder unsubstituiertes oder substituiertes Phenyl, den Rest

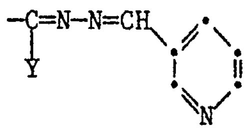

und
Y unsubstituierten oder substituierten aromatischen Carbo- oder Heterocyclus sowie die Salze einer Verbindung der Formel I.

2. Verbindungen der Formel Ia

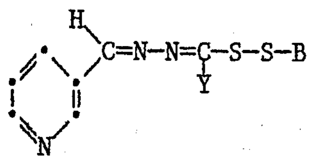

(Ia),

gemäss Anspruch 1, dadurch gekennzeichnet, dass
Y einen unsubstituierten oder substituierten aromatischen Carbo-oder Heterocyclus, und
B unsubstituiertes oder substituiertes $C_1$-$C_6$-Alkyl oder unsubstituiertes oder substituiertes Phenyl bedeuten, sowie die Salze einer Verbindung der Formel Ia.

3. Verbindungen gemäss Anspruch 2, dadurch gekennzeichnet, dass
Y Pyridyl oder unsubstituiertes oder substituiertes Phenyl
B $C_1$-$C_6$-Alkyl, ein- oder mehrfach halogeniertes $C_1$-$C_6$-Alkyl, $C_1$-$C_4$-Alkoxy-$C_1$-$C_6$-alkyl, $C_1$-$C_4$-Alkylthio-$C_1$-$C_6$-alkyl, Cyano-$C_1$-$C_6$-alkyl, Nitro-$C_1$-$C_6$-alkyl oder den Rest

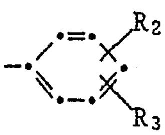

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeuten.

4. Verbindungen gemäss Anspruch 3, dadurch gekennzeichnet, dass Y Pyridyl oder den Rest

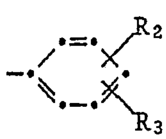

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeutet und B Cyano-$C_1$-$C_4$-alkyl bedeutet.

5. [1-(4-Chlorphenyl)-4-(pyrid-3-yl)-2,3-diazabutadien-1-yl]-(1,1-dimethyl-1-cyanomethyl) disulfid gemäss Anspruch 4.

6. Verbindungen gemäss Anspruch 3, dadurch gekennzeichnet, dass Y Pyridyl oder den Rest

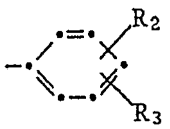

,

worin $R_2$ und $R_3$ voneinander unabhängig für Wasserstoff, Halogen, $C_1$-$C_4$-Alkyl, Trifluormethyl, $C_1$-$C_4$-Alkoxy oder Nitro steht, bedeutet und B Phenyl, Nitrophenyl, 4-Chlorphenyl oder 4-Tolyl bedeutet.

7. Verbindungen gemäss Anspruch 6, dadurch gekennzeichnet, dass Y 4-Chlorphenyl bedeutet.

8. Verbindungen der Formel Ib

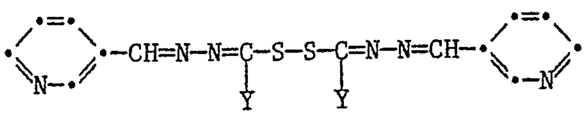

(Ib),

gemäss Anspruch 1, dadurch gekennzeichnet, dass Y einen Rest

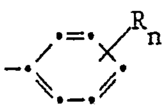

oder

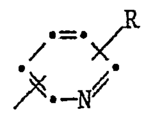

bedeutet, wobei
R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen, $C_1$-$C_4$-Halogenalkylthio mit 1 bis 9 Halogenatomen, Phenyl, substituiertes Phenyl, Phenyl-$C_1$-$C_3$-alkyl, Phenoxy, Phenylthio oder Pyridyloxy bedeutet und
n für eine Zahl von 1 bis 5 steht; sowie die Salze und optischen Isomeren einer Verbindung der Formel V.

9. Verbindungen gemäss Anspruch 8, dadurch gekennzeichnet, dass
R Wasserstoff, $C_1$-$C_4$-Alkyl, Halogen, Nitro, Cyano, $C_1$-$C_4$-Alkoxy, $C_1$-$C_4$-Alkylthio, $C_1$-$C_4$-Halogenalkyl mit 1 bis 9 Halogenatomen, $C_1$-$C_4$-Halogenalkoxy mit 1 bis 9 Halogenatomen oder $C_1$-$C_4$-Halogenalkylthio mit 1 bis 9 Halogenatomen bedeutet und n für eine Zahl von 1 bis 3 steht.

10. Verbindungen gemäss Anspruch 8 oder 9, dadurch gekennzeichnet, dass
R Wasserstoff, $C_1$-$C_4$-Alkyl, Fluor, Chlor, Brom, Nitro, $C_1$-$C_4$-Alkoxy oder Trifluormethyl bedeutet.

11. Verbindungen gemäss einem der Ansprüche 8 bis 10, dadurch gekennzeichnet, dass n für eine Zahl 1 oder 2 steht.

12. Verbindungen gemäss einem der Ansprüche 8 bis 11, dadurch gekennzeichnet, dass Y den Rest

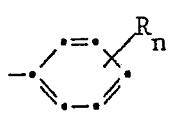

bedeuten, wobei sich mindestens ein R in 4-Stellung befindet.

13. Verbindungen gemäss Anspruch 12 der Formel

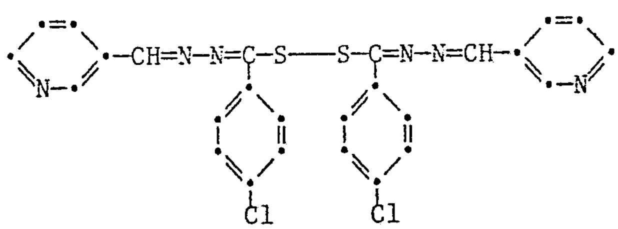

.

14. Verfahren zur Herstellung einer Verbindung der Formel Ia gemäss einem der Ansprüche 2-7, dadurch gekennzeichnet, dass man ein 5-Pyridyl-4,5-dihyrothiadiazol der Formel II

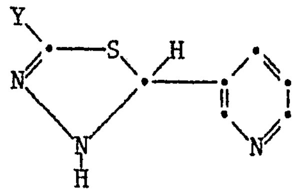

(II),

worin Y die unter einem der Ansprüche 2 bis 7 angegebenen Bedeutungen hat, zunächst mit einer Base, dann mit einem Sulfenylchlorid der Formel III,

Cl-S-B (III),

umsetzt, wobei Y und B die im Anspruch 1 angegebenen Bedeutungen haben, das erwünschte Produkt gegebenenfalls in eines seiner Salze überführt und isoliert.

15. Verfahren zur Herstellung einer Verbindung der Formel Ib gemäss einem der Ansprüchen 8 bis 13, dadurch gekennzeichnet, dass man eine Verbindung der Formel II, Anspruch 14,

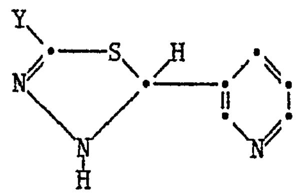

(II)

worin Y die unter einem der Ansprüche 8 bis 12 angegebenen Bedeutungen hat, oxidiert.

16. Schädlingsbekämpfungsmittel, welches als aktive Komponente eine Verbindung gemäss einem der Ansprüche 1 bis 13 zusammen mit geeigneten Trägern und/oder anderen Zuschlagstoffen enthält.

17. Verwendung einer Verbindung gemäss einem der Ansprüche 1 bis 13 zur Bekämpfung von Insekten und Vertretern der Ordnung Akarina an Tieren und Pflanzen.

18. Verwendung gemäss Anspruch 17 zur Bekämpfung von pflanzenschädigenden Insekten.

19. Verwendung gemäss Anspruch 18 zur Bekämpfung von pflanzenschädigenden saugenden Insekten.

20. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der Ansprüche 1 bis 13 oder mit einem Mittel enthaltend neben Zusatz- und Trägerstoffen eine pestizid wirksame Menge dieser Verbindung, in Kontakt bringt oder behandelt.